# EUROPEAN PATENT SPECIFICATION

(11) **EP 2 606 114 B1**
(45) Date of publication and mention of the grant of the patent: **01.04.2015**
(21) Application number: 11743558.6
(22) Date of filing: 16.08.2011
(51) Int. Cl.: C12C 7/24, C12C 7/26, C12N 9/52, C12C 5/00, C12H 1/00, C12G 3/00

(54) **A BREWING METHOD**
BRAUVERFAHREN
PROCÉDÉ DE BRASSAGE

(30) Priority: 17.08.2010 EP 10173035
(43) Date of publication of application: 26.06.2013
(73) Proprietor: Novozymes A/S, 2880 Bagsvaerd (DK)
(72) Inventor: KREISZ, Stefan, DK-2100 Copenhagen Oe (DK); BAEKGAARD, Lone, DK-2000 Frederiksberg (DK); FREDERIKSEN, Anne Mette Bhatia, DK-2100 Copenhagen Oe (DK); HOFF, Tine, DK-2840 Holte (DK); OESTERGAARD, Peter Rahbek, DK-2830 Virum (DK); OLSEN, Ole, DK-2300 Copenhagen S (DK); OLSSON, Ulf, S-24436 Kaevlinge (SE)
(74) Representative: Kofoed, Gertrud Sonne
(86) International application number: PCT/EP2011/064089
(87) International publication number: WO 2012/022745

(56) References cited:
- WO-A1-93/00925
- US-A- 4 038 419
- US-A- 5 035 902
- SIEBERT ET AL: "Mechanisms of Beer Colloidal Stabilization", JOURNAL OF THE AMERICAN SOCIETY OF BREWING CHEMISTS, AMERICAN SOCIETY OF BREWING CHEMISTS, ST PAUL, MN, US, vol. 55, no. 2, 1 January 1997 (1997-01-01), pages 73-78, XP002198641, ISSN: 0361-0470

### SEQUENCE LISTING

<110> Novozymes A/S
   Carlsberg Breweries A/S
   Kreisz, Stefan
   Baekgaard, Lone
   Frederiksen, Anne Mette Bhatia
   Hoff, Tine
   Oestergaard, Peter Rahbek
   Olsen, Ole
   Olsson, Ulf
<120> A BREWING METHOD
<130> 11765-WO-PCT
<160> 4
<170> PatentIn version 3.5
<210> 1
   <211> 136
   <212> PRT
   <213> Streptomyces violaceoruber
<220>
   <221> mat_peptide
   <222> (1)..(136)
<400> 1
   Ala Ala Ala Ala Val Thr Val Tyr Tyr Ser Ala Thr Asn Ala Pro Ser
   1               5                   10                  15
   Phe Arg Ser Gln Ile Ser Arg Ser Ala Gln Ile Trp Asn Ser Ser Val
               20                  25                  30
   Ser Asn Val Arg Leu Ala Glu Ser Ser Ser Gly Ala Asp Phe Ala Tyr
           35                  40                  45
   Tyr Glu Gly Asn Asp Ser Arg Gly Ser Tyr Ala Ser Thr Asp Gly His
       50                  55                  60
   Gly Ser Gly Tyr Ile Phe Leu Asp Tyr Arg Gln Asn Gln Gln Tyr Asp
   65                  70                  75                  80
   Ser Thr Arg Val Thr Ala His Glu Thr Gly His Val Leu Gly Leu Pro
                   85                  90                  95
   Asp His Tyr Ser Gly Pro Cys Ser Glu Leu Met Ser Gly Gly Gly Pro
               100                 105                 110
   Gly Pro Ser Cys Thr Asn Pro Tyr Pro Asn Ser Thr Glu Arg Ser Arg
           115                 120                 125
   Val Asn Gln Leu Trp Ala Tyr Gly
                       130                 135
<210> 2
   <211> 194
   <212> PRT
   <213> Kribbella flavida
<220>
   <221> mat_peptide
   <222> (1)..(194)
<400> 2
   Ala Pro Ala Pro Glu Arg Val Gln Ala Ala Thr Thr Thr Val Ser Ala
   1 5 10 15
   Tyr Ala Gly Ser Pro Gln Glu Ala Leu Asn Asn Lys Ala Phe Tyr Asp
   20 25 30
   Ala Val Met Lys Ser Ala Leu Ala Lys Gln Ala Lys Ala Gly Asn Arg
   35 40 45
   Leu Ala Ala Val Thr Val Arg Tyr Arg Val Asn Ala Pro Thr Phe Ala
   50 55 60
   Thr Gln Ile Ser Arg Ser Thr Gln Ile Trp Asn Ser Ser Val Thr Asn
   65 70 75 80
   Val Arg Leu Val Gln Ser Thr Ser Gly Tyr Asp Phe Tyr Tyr Thr Glu
   85 90 95
   Gly Asn Asp Pro Arg Gly Ser Tyr Ala Ser Thr Asn Gly His Gly Arg
   100 105 110
   Gly Tyr Ile Phe Leu Asp Tyr Ala Gln Ser Arg Val Tyr Asn Pro Leu
   115 120 125
   Arg Ile Thr Ala His Glu Thr Gly His Val Leu Gly Leu Pro Asp His
   130 135 140
   Tyr Ser Gly Pro Cys Ser Glu Leu Met Ser Gly Gly Gly Pro Gly Pro
   145 150 155 160
   Ser Cys Thr Asn Ala Tyr Pro Asn Ser Ala Glu Arg Ser Arg Val Asn
   165 170 175
   Gln Leu Trp Ala Asn Gly Phe Thr Gly Leu Ser Gly Thr Phe Lys Thr
   180 185 190
   Ile Arg
<210> 3
   <211> 325
   <212> PRT
   <213> Peptidase A1
<220>
   <221> mat_peptide
   <222> (1)..(325)
<400> 3
   Gly His Gly Thr Val Val Thr Ser Pro Glu Pro Asn Asp Ile Glu Tyr
   1               5                   10                  15
   Leu Thr Pro Val Asn Ile Gly Gly Thr Thr Leu Asn Leu Asp Phe Asp
               20                  25                  30
   Thr Gly Ser Ala Asp Leu Trp Val Phe Ser Glu Glu Leu Pro Lys Ser
           35                  40                  45
   Glu Gln Thr Gly His Asp Val Tyr Lys Pro Ser Gly Asn Ala Ser Lys
       50                  55                  60
   Ile Ala Gly Ala Ser Trp Asp Ile Ser Tyr Gly Asp Gly Ser Ser Ala
   65                  70                  75                  80
   Ser Gly Asp Val Tyr Gln Asp Thr Val Thr Val Gly Gly Val Thr Ala
                   85                  90                  95
   Gln Gly Gln Ala Val Glu Ala Ala Ser Lys Ile Ser Asp Gln Phe Val
               100                 105                 110
   Gln Asp Lys Asn Asn Asp Gly Leu Leu Gly Leu Ala Phe Ser Ser Ile
           115                 120                 125
   Asn Thr Val Lys Pro Lys Pro Gln Thr Thr Phe Phe Asp Thr Val Lys
       130                 135                 140
   Asp Gln Leu Asp Ala Pro Leu Phe Ala Val Thr Leu Lys Tyr His Ala
   145                 150                 155                 160
   Pro Gly Ser Tyr Asp Phe Gly Phe Ile Asp Lys Ser Lys Phe Thr Gly
                   165                 170                 175
   Glu Leu Ala Tyr Ala Asp Val Asp Asp Ser Gln Gly Phe Trp Gln Phe
               180                 185                 190
   Thr Ala Asp Gly Tyr Ser Val Gly Lys Gly Asp Ala Gln Lys Ala Pro
           195                 200                 205
   Ile Thr Gly Ile Ala Asp Thr Gly Thr Thr Leu Val Met Leu Asp Asp
       210                 215                 220
   Glu Ile Val Asp Ala Tyr Tyr Lys Gln Val Gln Gly Ala Lys Asn Asp
   225                 230                 235                 240
   Ala Ser Ala Gly Gly Tyr Val Phe Pro Cys Glu Thr Glu Leu Pro Glu
                   245                 250                 255
   Phe Thr Val Val Ile Gly Ser Tyr Asn Ala Val Ile Pro Gly Lys His
               260                 265                 270
   Ile Asn Tyr Ala Pro Leu Gln Glu Gly Ser Ser Thr Cys Val Gly Gly
           275                 280                 285
   Ile Gln Ser Asn Ser Gly Leu Gly Leu Ser Ile Leu Gly Asp Val Phe
       290                 295                 300
   Leu Lys Ser Gln Tyr Val Val Phe Asp Ser Gln Gly Pro Arg Leu Gly
   305                 310                 315                 320
   Phe Ala Ala Gln Ala
                   325
<210> 4
   <211> 177
   <212> PRT
   <213> Thermoascus aurantiacus
<220>
   <221> mat_peptide
   <222> (1)..(177)
<400> 4
   Thr Arg Ile Ser Ser Cys Ser Gly Ser Arg Gln Ser Ala Leu Thr Thr
   1               5                   10                  15
   Ala Leu Arg Asn Ala Ala Ser Leu Ala Asn Ala Ala Ala Asp Ala Ala
               20                  25                  30
   Gln Ser Gly Ser Ala Ser Lys Phe Ser Glu Tyr Phe Lys Thr Thr Ser
           35                  40                  45
   Ser Ser Thr Arg Gln Thr Val Ala Ala Arg Leu Arg Ala Val Ala Arg
       50                  55                  60
   Glu Ala Ser Ser Ser Ser Ser Gly Ala Thr Thr Tyr Tyr Cys Asp Asp
   65                  70                  75                  80
   Pro Tyr Gly Tyr Cys Ser Ser Asn Val Leu Ala Tyr Thr Leu Pro Ser
   85 90 95
   Tyr Asn Ile Ile Ala Asn Cys Asp Ile Phe Tyr Thr Tyr Leu Pro Ala
   100 105 110
   Leu Thr Ser Thr Cys His Ala Gln Asp Gln Ala Thr Thr Ala Leu His
   115 120 125
   Glu Phe Thr His Ala Pro Gly Val Tyr Ser Pro Gly Thr Asp Asp Leu
   130 135 140
   Ala Tyr Gly Tyr Gln Ala Ala Met Gly Leu Ser Ser Ser Gln Ala Val
   145 150 155 160
   Met Asn Ala Asp Thr Tyr Ala Leu Tyr Ala Asn Ala Ile Tyr Leu Gly
   165 170 175
   Cys

## Description

### FIELD OF THE INVENTION

This application is related to a method of brewing wherein a snapalysin is used for managing the colloidal stability of beverages. The snapalysin may be obtainable from Actinomycetales. The enzyme is added either to the mash and/or to the wort during the production of beer.

### BACKGROUND OF THE INVENTION

Many beverages like beer, wine, juice etc. develop precipitates during manufacture or upon storage. This phenomenon is described as haze formation. One form of haze formation is generally believed to be due to interaction of proteins and polyphenols present in the beverage. This interaction leads to the formation of insoluble or semi-soluble suspension of colloidal particles. Since haze formation may resemble cloudiness produced by microbial contamination, it is generally preferred that the beverages, particularly beer, are very clear and transparent even upon long storage. Hence processes have been developed to reduce such haze formation. These processes target either the proteins or the polyphenols or both.

Silica gels, Bentonite, Poly(VinylPolyPyrrolidone) (PVPP) etc. have been used to adsorb proteins and polyphenols, decreasing haze formation and improving colloidal stability. However such materials, when used repeatedly result in diminishing returns and consequently lead to increased costs. Moreover, they also remove other desirable compounds from the beverage, which may affect its quality.

Enzymes, particularly proteases, are also used during fermentation to improve the colloidal stability of beverages, particularly beer. Traditionally, proteases like papain and bromelain have been used to reduce chill haze formation. However these proteases have been shown to affect the foam stability of the beverage by hydrolysing the proteins that are involved in formation and stabilization of foam. Moreover these also cause flavour changes in the beverage. Another approach has been by the use of proteases that hydrolyse mostly the haze forming proteins and rarely the foam forming proteins. For example, a prolyl specific endoprotease, added during fermentation, is known, e.g., from EP 1326957, which supposedly hydrolyses the haze forming proteins thus improving colloidal stability and retaining foam stability. Use of proteases during fermentation might retain their activity in the final beer which is generally undesirable. Inactivation by pasteurization can furthermore lead to a burned flavour in the beer. However pasteurization is also generally recommended to assure microbial stability.

US 5035902 discloses a foam stabilizing proteinase preparation from Candida.

There still exists a need for improved processes for colloidal stabilization of beverages, especially beer.

### SUMMARY OF THE INVENTION

In one aspect, the invention relates to a method of improving colloidal stability in beer comprising contacting a mash and/or a wort with a snapalysin during the production of beer.

In another aspect, the invention relates to the use of snapalysin in brewing.

In another aspect, the invention relates to the use of snapalysin in brewing of beer.

In another aspect, the contacting is done with the wort.

In another aspect, the snapalysin is added to the wort.

In another aspect, the contacting is done with the mash.

In another aspect, the snapalysin is obtainable from Actinomycetales.

In another aspect, the Actinomycetale belongs to the genus *Streptomyces.*

In another aspect, the Actinomycetale belongs to the genus *Kribbella.*

In another aspect, the contacting is done from 5 minutes to 120 minutes.

In another aspect, about 0.01 to about 100 mg snapalysin EP (Enzyme Protein) per kg malt is used.

In another aspect, about 0.0016 to about 16 mg snapalysin EP per litre of wort is used.

In another aspect, using the method of the invention, the colloidal stability is increased by at least 10% compared to a beer brewed in the absence of snapalysin.

In another aspect, using the method of the invention, the foam stability is at least 80% compared to a beer brewed in the absence of snapalysin.

In another aspect, the method results in a decreased or zero use of processing aids when compared to a beer brewed in a conventional way.

In another aspect, the beer is produced without processing aids.

In another aspect, a beer is produced without stabilisation with silica-gel.

In another aspect, the contacting is done after lautering.

In another aspect, the contacting is done during sparging.

In another aspect, the contacting is done at a temperature in the range of from 20° to 80°C.

In another aspect, the contacting is done at a temperature of at least 30°C.

In another aspect, the contacting is done at a temperature of at least 40°C.

In another aspect, the contacting is done at a temperature of at least 50°C.

In another aspect, the contacting is done at a temperature of at least 60°C.

In another aspect, the contacting is done at a temperature of at least 70°C.

In another aspect, the contacting is done at a temperature of at least 75°C.

### DETAILED DESCRIPTION OF THE INVENTION

The process of beer-brewing is well known to the person skilled in the art. A conventional procedure may be outlined in the following way: The starting material is malted (i.e. dampened or soaked, germinated and subsequently dried) barley and/or unmalted adjuncts, called the grist. During the mashing, where the grist is ground and mixed with water, heated and stirred, the polymers including carbohydrates are degraded by the aid of the enzymes naturally present in the malt and also exogenously added enzymes, if any. After mashing, it is necessary to separate the liquid extract (the wort) from the solids (spent grain particles and adjunct particles) in order to get clear wort. This process is described as lautering. Prior to lautering, the mash temperature may be raised to about 75-78°C (known as mashing-off). Wort filtration is important because the solids are enriched in large amounts of protein, poorly modified starch, lipids and fatty acids, silicates, and polyphenols (tannins) and proteins. The extract retained in the spent grain after collection of the first wort may also be washed out by adding hot water on top of the lauter cake. This process is called sparging. The hot water flows through the spent grain and dissolves the remaining extract. The diluted wort is called second wort and its extract decreases from the original gravity of the first wort down to 1-2 %. After addition of hops, the wort is boiled. Hereby numerous substances including several proteins are denatured and a precipitation of protein-polyphenol complexes will take place. After cooling and removal of precipitates, the finished beer wort is aerated and yeast is added. After a main fermentation, lasting typically 5-10 days, most of the yeast is removed and the so-called green beer is stored at a low temperature, typically at 0 - 5°C for 1 to 12 weeks. During this period the remaining yeast will precipitate or sediment together with protein-polyphenol complexes and other insoluble substances. To remove the remaining excess dispersed particles, a filtration is performed. The fermented beer may now be carbonized prior to bottling. Carbon dioxide not only contributes to the perceived "fullness" or "body", it imparts "tingling" and "freshness" too. Moreover it acts as a flavor enhancer, and as an enhancer of the foaming potential and plays an important role in extending the shelf life of the product.

The term "beer" as used herein is intended to cover at least beer prepared from mashes prepared from unmalted cereals as well as all mashes prepared from malted cereals, and all mashes prepared from a mixture of malted and unmalted cereals. The term "beer" also covers beers prepared with adjuncts, and beers with all possible alcohol contents.

Without being bound by theory, it is believed that the interaction between proteins and polyphenols in beer stored at low temperatures or for a long time, leads to development of aggregates which are referred to as haze. Since the formation of haze affects one of the quality parameters of beer i.e.,colloidal stability, methods have been developed which prevent such haze formation. During the process of brewing, a majority of the protein-polyphenol complexes precipitate by cooling the liquid during beer maturation. Any remaining polyphenols and/or proteins are removed using PVPP, silica gel, bentonite etc.

Colloidal stability of a beer may be defined as the amount of warm cycles before the colloidal instability measured in EBC units becomes greater than 2. One warm cycle corresponds to approximately 25 days of shelf-life (MEBAK 2.15.2.1, Fociertmethode, Vorausbestimmung der chemisch-physikalischen Stabilität, Methodensamlung der Mitteleuropaischen Brautechniche Analysekommission (MEBAK), Selbstverlag der MEBAK, Freising Weihenstephan). The measurement is done as detailed in Example 3. For purposes of this invention, colloidal stability can also be measured by analysing the amount of haze sensitive proteins (Analytica-EBC: Beer-9.40) with Brewtan C (a gallotannin), using methods as detailed in Example 1.

Haze is also referred to as "cloudiness" or "turbidity" or "colloidal instability" in the art and hence can be used interchangeably.

Another method of reducing haze formation is by the use of proteases.

Proteases like papain, are used to cleave the proteins, possibly yielding lower and/or more soluble protein-polyphenol aggregates. However the use of these enzymes also affects the proteins involved in foam formation and foam stability, further affecting the quality of the final beer.

The inventors have surprisingly found that contacting a mash and/or a wort with a snapalysin during the production of beer leads to improved colloidal stabilization and/or foam stability.

The inventors have also surprisingly found that contacting a mash and/or a wort with a snapalysin during the production of beer leads to improved colloidal stabilization without substantially affecting foam stability.

The terms "mash", and "wort" have the conventional meaning in the art.

Snapalysins (EC 3.4.24.77) are small neutral metallo-proteases belonging to the M7 protease family. They are also called peptidases of the M7 family according to the MEROPS classification (http://merops.sanger.ac.uk/). Snapalysins are known in the art. For example, see, Butler, M.J. Snapalysin In: Barrett, A.J., Rawlings, N.D. and Woessner, J.F. (Eds.) Handbook of Proteolytic Enzymes, Academic Press, London, 1998, 1134-1135

In one aspect, the snapalysin is obtainable from Actinomycetales.

In one aspect, the actinomycetale is *Streptomyces.*

Actinomycetales are known in the art. They are organisms belonging to the phylum Actinobacteria, class Actinobacteria, subclass Actinobacteridae, and order Actinomycetales.

*Streptomyces* are Actinomycetales belonging to suborder Streptomycineae, family Streptomycetaceae and genus Streptomyces.

Various species of *Streptomyces* genus have been identified, including but not limited to, *Streptomyces coelicolor, S. lividans, S. albicans, S. griseus, S. plicatosporus, S. violaceoruber* etc.

In a preferred aspect, the snapalysin is obtainable from *S. violaceoruber.*

In another preferred aspect, the snapalysin is obtainable from *S. lividans.*

In one aspect, the actinomycetale is *Kribbella.*

*Kribbella* are actinomycetales belonging to suborder Propionibacterineae, family Nocardioidaceae and genus *Kribbella.*

In a preferred aspect, the snapalysin is obtainable from *Kribbella flavida.*

The term "obtainable from" as used herein in connection with a given source shall mean that the polypeptide encoded by the nucleic acid sequence is produced by the source or by a recombinant cell (also called a host cell) in which the nucleic acid sequence from the source is present. In a preferred embodiment, the polypeptide is secreted extracellularly. In another preferred embodiment, the polypeptide is intracellular. Depending upon the host employed in a recombinant production procedure, the snapalysins of the present invention may be glycosylated or may be non-glycosylated. In addition, the snapalysins of the invention may also include an initial modified methionine residue, in some cases as a result of host-mediated processes.

The host cells may be a unicellular microorganism, e.g., a prokaryote, or a non-unicellular microorganism, e.g., a eukaryote.

Useful host cells are bacterial cells such as gram positive bacteria including, but not limited to, a *Bacillus* cell, or a *Streptomyces* cell, or cells of lactic acid bacteria; or gram negative bacteria such as *E. coli* and *Pseudomonas* sp. Lactic acid bacteria include, but are not limited to, species of the genera *Lactococcus, Lactobacillus, Leuconostoc, Streptococcus, Pediococcus,* and *Enterococcus.*

The prokaryotic host cell may be any Gram-positive or Gram-negative bacterium. Gram-positive bacteria include, but are not limited to, *Bacillus, Clostridium, Enterococcus, Geobacillus, Lactobacillus, Lactococcus, Oceanobacillus, Staphylococcus, Streptococcus,* and *Streptomyces.* Gram-negative bacteria include, but are not limited to, *Campylobacter, E. coli, Flavobacterium, Fusobacterium, Helicobacter, Ilyobacter, Neisseria, Pseudomonas, Salmonella,* and *Ureaplasma.*

The bacterial host cell may be any *Bacillus* cell including, but not limited to, *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* and *Bacillus thuringiensis* cells.

The bacterial host cell may also be any *Streptococcus* cell including, but not limited to, *Streptococcus equisimilis, Streptococcus pyogenes, Streptococcus uberis,* and *Streptococcus equi* subsp. *Zooepidemicus* cells.

The bacterial host cell may also be any *Streptomyces* cell including, but not limited to, *Streptomyces achromogenes, Streptomyces avermitilis, Streptomyces coelicolor, Streptomyces griseus,* and *Streptomyces lividans* cells.

The introduction of DNA into a *Bacillus* cell may be effected by protoplast transformation (see, *e.g.,* Chang and Cohen, 1979, Mol. Gen. Genet. 168: 111-115), competent cell transformation (see, e.g., Young and Spizizen, 1961, J. Bacteriol. 81: 823-829, or Dubnau and Davidoff-Abelson, 1971, J. Mol. Biol. 56: 209-221), electroporation (see, *e.g.,* Shigekawa and Dower, 1988, Biotechniques 6: 742-751), or conjugation (see, e.g., Koehler and Thorne, 1987, J. Bacteriol. 169: 5271-5278). The introduction of DNA into an *E. coli* cell may be effected by protoplast transformation (see, *e.g.,* Hanahan, 1983, J. Mol. Biol. 166: 557-580) or electroporation (see, *e.g.,* Dower et al., 1988, Nucleic Acids Res. 16: 6127-6145). The introduction of DNA into a *Streptomyces* cell may be effected by protoplast transformation, electroporation (see, *e.g.,* Gong et al., 2004, Folia Microbiol. (Praha) 49: 399-405), conjugation (see, *e.g.,* Mazodier et al., 1989, J. Bacteriol. 171: 3583-3585), or transduction (see, e.g., Burke et a/., 2001, Proc. Natl. Acad. Sci. USA 98: 6289-6294). The introduction of DNA into a *Pseudomonas* cell may be effected by electroporation (see, *e.g.,* Choi et al., 2006, J. Microbiol. Methods 64: 391-397) or conjugation (see, *e.g.,* Pinedo and Smets, 2005, Appl. Environ. Microbiol. 71: 51-57). The introduction of DNA into a *Streptococcus* cell may be effected by natural competence (see, *e.g.,* Perry and Kuramitsu, 1981, Infect. Immun. 32: 1295-1297), protoplast transformation (see, *e.g.*, Catt and Jollick, 1991, Microbios 68: 189-207), electroporation (see, *e.g.,* Buckley et a/., 1999, Appl. Environ. Microbiol. 65: 3800-3804), or conjugation (see, *e.g.,* Clewell, 1981, Microbiol. Rev. 45: 409-436). However, any method known in the art for introducing DNA into a host cell can be used.

The host cell may also be a eukaryote, such as a mammalian, insect, plant, or fungal cell.

The host cell may be a fungal cell. "Fungi" as used herein includes the phyla Ascomycota, Basidiomycota, Chytridiomycota, and Zygomycota as well as the Oomycota and all mitosporic fungi (as defined by Hawksworth et al., In, Ainsworth and Bisby's Dictionary of The Fungi, 8th edition, 1995, CAB International, University Press, Cambridge, UK).

The fungal host cell may be a yeast cell. "Yeast" as used herein includes ascosporogenous yeast (Endomycetales), basidiosporogenous yeast, and yeast belonging to the Fungi Imperfecti (Blastomycetes). Since the classification of yeast may change in the future, for the purposes of this invention, yeast shall be defined as described in Biology and Activities of Yeast (Skinner, Passmore, and Davenport, editors, Soc. App. Bacteriol. Symposium Series No. 9, 1980).

The yeast host cell may be a *Candida, Hansenula, Kluyveromyces, Pichia, Saccharomyces, Schizosaccharomyces,* or *Yarrowia* cell, such as a *Kluyveromyces lactis, Saccharomyces carlsbergensis, Saccharomyces cerevisiae, Saccharomyces diastaticus, Saccharomyces douglasii, Saccharomyces kluyveri, Saccharomyces norbensis, Saccharomyces oviformis,* or *Yarrowia lipolytica* cell.

The fungal host cell may be a filamentous fungal cell. "Filamentous fungi" include all filamentous forms of the subdivision Eumycota and Oomycota (as defined by Hawksworth *et al*., 1995, *supra*). The filamentous fungi are generally characterized by a mycelial wall composed of chitin, cellulose, glucan, chitosan, mannan, and other complex polysaccharides. Vegetative growth is by hyphal elongation and carbon catabolism is obligately aerobic. In contrast, vegetative growth by yeasts such as *Saccharomyces cerevisiae* is by budding of a unicellular thallus and carbon catabolism may be fermentative.

The filamentous fungal host cell may be an *Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, SchizophylJum, Talaromyces, Thermoascus, Thielavia, Tolypocladium, Trametes,* or *Trichoderma* cell.

For example, the filamentous fungal host cell may be an *Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merrtarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Thielavia terrestris, Trametes villosa, Trametes versicolor, Trichoderma harzianum, Trichoderma koningii, Tiichoderma longibrachiatum, Trichoderma reesei,* or *Trichoderma viride* cell.

Fungal cells may be transformed by a process involving protoplast formation, transformation of the protoplasts, and regeneration of the cell wall in a manner known *per se.* Suitable procedures for transformation of *Aspergillus* and *Trichoderma* host cells are described in EP 238023, Yelton et al., 1984, Proc. Natl. Acad. Sci. USA 81: 1470-1474, and Christensen et al., 1988, BiolTechnology 6: 1419-1422. Suitable methods for transforming *Fusarium* species are described by Malardier et al., 1989, Gene 78: 147-156, and WO 96/00787. Yeast may be transformed using the procedures described by Becker and Guarente, In Abelson, J.N. and Simon, M.I., editors, Guide to Yeast Genetics and Molecular Biology, Methods in Enzymology, Volume 194, pp 182-187, Academic Press, Inc., New York; Ito et al., 1983, J. Bacteriol. 153: 163; and Hinnen et al., 1978, Proc. Natl. Acad. Sci. USA 75: 1920.

In the production methods of the present invention, the cells are cultivated in a nutrient medium suitable for production of snapalysin using methods known in the art. For example, the cell may be cultivated by shake flask cultivation, small-scale or large-scale fermentation (including continuous, batch, fed-batch, or solid state fermentations) in laboratory or industrial fermentors performed in a suitable medium and under conditions allowing the polypeptide to be expressed and/or isolated. The cultivation takes place in a suitable nutrient medium comprising carbon and nitrogen sources and inorganic salts, using procedures known in the art. Suitable media are available from commercial suppliers or may be prepared according to published compositions (e.g., in catalogues of the American Type Culture Collection). If snapalysin is secreted into the nutrient medium, it can be recovered directly from the medium. If the snapalysin is not secreted, it can be recovered from cell lysates.

The resulting snapalysin may be recovered by methods known in the art. For example, the snapalysin may be recovered from the nutrient medium by conventional procedures including, but not limited to, centrifugation, filtration, extraction, spray-drying, evaporation, or precipitation.

The snapalysins of the present invention may be purified by a variety of procedures known in the art including, but not limited to, chromatography (e.g., ion exchange, affinity, hydrophobic, chromatofocusing, and size exclusion), electrophoretic procedures (e.g., preparative isoelectric focusing), differential solubility (e.g., ammonium sulfate precipitation), SDS-PAGE, or extraction (see, e.g., Protein Purification, J.-C. Janson and Lars Ryden, editors, VCH Publishers, New York, 1989) to obtain substantially pure polypeptides. In an alternative aspect, the snapalysin is not recovered, but rather a host cell of the present invention expressing the snapalysin is used as a source of snapalysin.

A snapalysin to be used according to the invention is preferably purified. The term "purified" as used herein covers enzyme protein preparations where the preparation has been enriched for the enzyme protein in question. Such enrichment could for instance be: the removal of the cells of the organism from which an enzyme protein was produced, the removal of non-protein material by a protein specific precipitation or the use of a chromatographic procedure where the enzyme protein in question is selectively adsorbed and eluted from a chromatographic matrix. The snapalysin may have been purified to an extent so that only minor amounts of other proteins are present. The expression "other proteins" relates in particular to other enzymes. A snapalysin to be used in the method of the invention may be "substantially pure", i.e. substantially free from other components from the organism in which it was produced, which may either be a naturally occurring microorganism or a genetically modified host microorganism for recombinant production of the snapalysin.

However, for the uses according to the invention, the snapalysin need not be that pure. It may, e.g., include other enzymes.

In a preferred aspect, the snapalysin to be used in the method of the invention has been purified to contain at least 20%, preferably at least 30%, at least 40% or at least 50%, (w/w) of snapalysin out of the total protein.

In another embodiment of the methods of the invention, the snapalysin is recombinantly produced.

In another aspect, contacting of the snapalysin is with either a mash or a wort or both.

In another aspect, the contacting of the snapalysin is with the mash or with the mashing water or with the grist.

In another aspect, the contacting with the mash is during mashing.

In another aspect, the contacting with the mash is during mashing-off.

In another aspect the contacting is during lautering.

In another aspect the contacting is during sparging.

In another aspect, the contacting is with the wort.

In another aspect, the snapalysin is added to the wort.

In another aspect the contacting is after lautering.

In another aspect, the contacting is after lautering but before wort boiling.

The snapalysin may be used alone or preferably in the form of an enzyme composition. Such enzyme compositions are known to a person skilled in the art. Enzyme composition may also optionally contain other enzymes and/or other stabilizers that help stabilize the enzyme(s). The compositions of the invention may be in any form suited for the use in question, e.g. in the form of a dry powder or granulate, in particular a non-dusting granulate, a liquid, in particular a stabilized liquid, an immobilized form or a protected enzyme. Granulates may be produced, e.g. as disclosed in U.S. Pat. No. 4,106,991 and U.S. Pat. No. 4,661,452 (both to Novo Industri A/S), and may optionally be coated by methods known in the art. Liquid enzyme preparations may, for instance, be stabilized by adding nutritionally acceptable stabilizers such as a sugar, a sugar alcohol or another polyol, lactic acid or another organic acid according to established methods. Protected enzymes may be prepared according to the method disclosed in EP 238,216.

The contacting is done at a temperature depending on the optimum temperature for the enzyme and also the stage at which the enzyme is added. The skilled person would know how to calculate the optimum temperature for the enzyme. For purposes of this invention the contacting is done generally at temperature of at least 20 °C, e.g., at least 25 °C, at least 30 °C, at least 35 °C, at least 40 °C, at least 45 °C at least 50 °C, at least 55 °C preferably such as at least 60 °C, such as at least 65 °C, more preferably such as at least 70 °C and most preferably such as 75-80 °C. In particular, the contacting is done at a temperature range of about 20-80 °C, e.g. 30-80 °C, such as about 40-80 °C, preferably such as about 50-80 °C.

In one aspect, the contacting is done for a period between 3 min to 5 hours, e.g. between 5 min to 5 hours, preferably between 5 min and 4 hours, more preferably between 5 min to 180 min e.g., between 5 min to 120 min, more preferably between 10 min and 120 min and most preferably between 30 min and 90 min.

The amount of enzyme used for contacting generally depends on various factors for example but not limited to the type of enzyme, the activity of the enzyme etc. For purposes of this invention, the amount of enzyme used will generally be about 0.01 mg to about 100 mg snapalysin EP (Enzyme Protein) per kg of the substrate, preferably about 0.05 to about 50 mg EP/kg of the substrate, more preferably about 0.1 to about 40 mg EP per kg of the substrate.

In particular, the amount of enzyme used will generally be about 0.01 mg to about 100 mg snapalysin EP (Enzyme Protein) per kg of the malt, preferably about 0.05 to about 50 mg EP/kg of the malt, more preferably about 0.1 to about 40 mg EP per kg of the malt.

The enzyme can also be added to a liquid substrate and in such cases, the amount of enzyme used will generally be about 0.0016 mg to about 16 mg snapalysin EP per litre of the substrate, preferably about 0.008 mg to about 8 mg EP per litre of the substrate, more preferably about 0.016 mg to about 6.4 mg EP per litre of the substrate.

In particular, the amount of enzyme used will generally be about 0.0016 mg to about 16 mg snapalysin EP per litre of the wort, preferably about 0.008 mg to about 8 mg EP per litre of the wort, more preferably about 0.016 mg to about 6.4 mg EP per litre of the wort.

The snapalysin activity may be measured using any protease assay, in which a substrate is employed, that includes peptide bonds relevant for the specificity of the protease in question. Assay-pH and assay-temperature are likewise to be adapted to the protease in question. Examples of assay-pH-values are pH 2, 3, 4, 5, 6, 7, 8, 9, 10, 11, or 12. Examples of assay-temperatures are 30, 35, 37, 40, 45, 50, 55, 60, 65, 70, 80, 90, or 95 °C. Examples of protease substrates include but are not limited to casein, such as Azurine-Crosslinked Casein (AZCL-casein) and Protazyme AK.

In one aspect, using the method of the invention, the colloidal stability is increased by at least 10% e.g. at least 15%, at least 20%, at least 25%, at least 30%, at least 35%, at least 40%, at least 45%, at least 50%, at least 55%, at least 60%, at least 65%, at least 70%, at least 75% such as at least 80%, such as at least 81%, such as at least 82%, such as at least 83%, such as at least 84%, such as at least 85% such as at least 86%, such as at least 87%, such as at least 88%, such as at least 89%, such as at least 90%, such as at least 91%, such as at least 92%, such as at least 93%, such as at least 94%, such as at least 95% such as at least 96%, such as at least 97%, such as at least 98%, such as at least 99%, such as at least 100%, such as at least 101%, such as at least 102%, such as at least 103%, such as at least 104%, such as at least 105% such as at least 106%, such as at least 107%, such as at least 108%, such as at least 109%, such as at least 110% compared to a beer brewed in the absence of snapalysin. In another aspect, the colloidal stability is increased in the range of about 10-110%, e.g. about 20-110%, 30-110%, 40-110%, preferably about 50-110%, more preferably in the range of 60-110%, most preferably in the range of 70-110%, even most preferably in the range of 80-110% compared to a beer brewed in the absence of snapalysin.

Colloidal stability may be measured, for example, by use of the method as described in Example 3.

In one aspect, the method of the invention leads to a reduction in haze, when compared to a beer brewed in the absence of snapalysin.

In one aspect, the haze is reduced by at least 5 %, e.g., at least 10%, at least 15%, at least 20%, at least 25%, at least 30%, at least 35%, at least 40%, at least 45%, at least 50%, at least 55%, at least 60%, at least 65%, at least 70%, at least 75%, such as at least 80%, such as at least 81%, such as at least 82%, such as at least 83%, such as at least 84%, such as at least 85% such as at least 86%, such as at least 87%, such as at least 88%, such as at least 89%, such as at least 90%, such as at least 91%, such as at least 92%, such as at least 93%, such as at least 94%, such as at least 95% such as at least 96%, such as at least 97%, such as at least 98%, such as at least 99%, such as at least 100% when compared to a beer brewed in the absence of snapalysin. In another aspect, the haze is reduced in the range of about 5-100%, e.g. about 10-100%, 30-100%, 40-100%, preferably about 50-100%, more preferably in the range of 60-100%, most preferably in the range of 70-100%, even most preferably in the range of 80-100% compared to a beer brewed in the absence of snapalysin.

In another aspect, the method of the invention leads to a reduction of haze when compared to a beer processed by using a processing aid.

In one aspect, the haze is reduced by at least 5 %, e.g., at least 10%, at least 15%, at least 20%, at least 25%, at least 30%, at least 35%, at least 40%, at least 45%, at least 50%, at least 55%, at least 60%, at least 65%, at least 70%, at least 75%, such as at least 80%, such as at least 81%, such as at least 82%, such as at least 83%, such as at least 84%, such as at least 85% such as at least 86%, such as at least 87%, such as at least 88%, such as at least 89%, such as at least 90%, such as at least 91%, such as at least 92%, such as at least 93%, such as at least 94%, such as at least 95% such as at least 96%, such as at least 97%, such as at least 98%, such as at least 99%, such as at least 100% when compared to a beer processed by using a processing aid. In another aspect, the haze is reduced in the range of about 5-100%, e.g. about 10-100%, about 20-100%, 30-100%, 40-100%, preferably about 50-100%, more preferably in the range of 60-100%, most preferably in the range of 70-100% , even most preferably in the range of 80-100% compared to a beer processed by using a processing aid.

To quantify the amount of haze in a beverage, a turbidimeter also called a hazemeter is often used. In a turbidimeter the amount of light is measured that is scattered at a pre-described angle relative to the direction of the incident light beam. Turbidity measurements are very suitable for the measurement of haze formed as the result of protein-polyphenol interactions.

The haze may be measured, for example, by using of one of methods as described in materials and methods.

In another aspect, the method of the invention leads to a decreased use of the processing aids used during brewing and storage to reduce the haze formation.

A "processing aid" is an agent that is used during brewing and/or storage to reduce the haze formation. The processing aids include but are not limited to for e.g. silica gel, PVPP, bentonite.

In one aspect, the decrease is 100 percent, meaning no processing agents are used.

In another aspect, the beer is produced without stabilization with silica and preferably without stabilization with silica and PVPP.

In one aspect, using the method of the invention, the foam stability is not affected compared to a beer brewed in the absence of snapalysin.

In one aspect, using the method of the invention, the foam stability is not affected compared to a beer processed using a processing aid.

In one aspect, the method of the invention leads to a beer that has a foam stability of at least 80% compared to the foam stability of beer brewed in the absence of snapalysin.

In another aspect, the method of the invention leads to a beer that has a foam stability of at least 80% when compared to a beer processed using a processing aid.

Foam is formed by the carbon dioxide released due to release of pressure during dispensing of beer. The foam is made stable due to the presence of surface active agents like foam active proteins which collect on the bubble surface and form an elastic skin around the gas bubble.

Foam stability of a beer may be measured, for example, by a use of a method as in example 1.

In one aspect, the foam stability of the beer is at least 80%, e.g., 81%, 82%, 83%, 84%, such as at least 85%, e.g., 86%, 87%, 88%, 89%, such as at least 90%, e.g., 91%, 92%, 93%, 94%, such as at least 95%, e.g., 96%, 97%, 98%, such as at least 99% compared to a beer brewed in the absence of snapalysin. In another aspect, the foam stability of the beer is in the range between 80-99%, e.g., 85-99%, 90-99%, 95-99% compared to a beer brewed in the absence of snapalysin.

In another aspect, the foam stability of the beer is at least 80%, e.g., 81%, 82%, 83%, 84%, such as at least 85%, e.g., 86%, 87%, 88%, 89%, such as at least 90%, e.g., 91%, 92%, 93%, 94%, such as at least 95%, e.g., 96%, 97%, 98%, such as at least 99% compared to a beer brewed in the absence of processing aids. In another aspect, the foam stability of the beer is in the range between 80-99%, e.g., 85-99%, 90-99%, 95-99% compared to a beer brewed in the absence of processing aids.

In one aspect, the invention relates to the use of snapalysin in brewing, particularly brewing of beer.

In another aspect, the invention relates to a process employing snapalysin in brewing, particularly brewing of beer. In another aspect, the invention relates to a brewing process employing snapalysin.

### MATERIALS AND METHODS:

Malt and other brewing materials used were of commercial grade. Chemicals used as buffers and substrates were commercial products of at least reagent grade. The enzymes were either made internally using standard recombinant techniques or commercially available from Novozymes A/S (Bagsvaerd, Denmark).

### Preparation of wort and beer:

### 2L brewing trials:

In pilot scale, mashing trials with 100 % malt were executed. Following the mashing profile in Table 1, 200L total wort volume using approximately 50 kg malt was produced and CaCl₂ and ZnCl₂ were added in appropriate amounts. Two times 2L wort were separated after lautering, °Plato was ensured to be between 13-14 and the temperature was adjusted to the indicated temperature (°C). pH was adjusted to a given pH, when indicated. Thereafter one aliquot of the wort was incubated with the enzyme at the indicated amount for 1 hour. The control was also kept for 1 hour at the same temperature without enzyme treatment.

**Table 1: mashing profile**

| Time (min) | Temp (°C) |
|---|---|
| 0 | 60 |
| 5 | 64 |
| 50 | 64 |
| 60 | 72 |
| 70 | 72 |
| 75 | 78 |
| 80 | 78 |

After the enzyme treatment, the wort was boiled, hops were added, and thereafter cooled to 15 °C. It was then fermented using a bottom fermenting yeast strain for 7 days at 15°C. Finally the beer was filtered.

### 10L Pilot brew:

Standard wort similar to the one described above with ~13 °Plato was made. Two times 10L wort were separated after lautering and both temperature and pH were adjusted to the indicated values. Thereafter one aliquot of the wort was incubated with the indicated amount of enzyme at the indicated pH and temperature for 1 hour. The control was also kept for 1 hour at the same pH and temperature without enzyme treatment. After the enzyme treatment, hops were added to the wort and subsequently boiled for 60 min, followed by a standard fermentation. Finally, the beer was filtered.

### Measurement of Haze:

### 2L lab brewing trials.

The haze for the beer, was measured using the following method. 200 microliter beer (filtered, degassed and pH adjusted (pH 4.0)) was added to a microtiter plate. A measurement was done (PolarStar OPTIMA plate reader, A550nm) to determine the background value. 100 microliter Brewtan C (200 mg/L) was then added to make up the volume to 300 microliter. The sample was analyzed with the plate reader every 2 min for a total of 16 minutes. The background value was subtracted from the measurement. The resulting value is a measurement of the content of haze sensitive proteins in the beer. The analysis was made with quadruple samples and then an average was calculated from the four measurements.

The haze in beer can also alternatively be measured by a modified method according to the method published by Siebert 1997 (J. Am. Soc. Brew. Chem. 55(2):73-78, 1997). 100 ml beer (degassed and pH adjusted (pH 4.0)) and 100 ml water were added in a glass with a magnetic stirrer and the amount of background haze value was determined with a Haze-meter (Haze-meter HZ-013, Lg-automatic APS, Denmark). 2x3.75 ml of 200 mg/L Brewtan C was added to the sample during stirring and the sample was incubated at room temperature for 40 minutes before measuring the developed haze. The background value was then subtracted from the measurement after 40 minutes to give a measure of the potential of forming haze in the final fermented beer.

### 10L Pilot brew:

Haze was measured by a forced aging method (0/40/0°C) (MEBAK II 2.15.2.1), in which beers were incubated in 24 h-cycles of 0°C and 40°C, respectively to accelerate haze. The formed haze was measured at a scattering angle of 90°.

### Measurement of Foam:

### 2L lab brewing trials.

200 ml beer (degassed and pH adjusted (pH 4.0) was added to a foam tower. Nitrogen was bubbled through a glass filter in the foam tower. The foam formed by the bubbling of nitrogen was then transported in tubes to a graded (ml) collector funnel which was resting on a scale.

The graded funnel also enables the measurement of collapsed beer volume. A scale on which the funnel is resting enables the resulting foam weight to be recorded. The analysis gave the weight of formed foam (g). The total weight (g) of the foam was put against the time (min) in a graph.

### 10L Pilot brew:

Foam stability was measured with a NIBEM foam tester according to MEBAK II 2.19.2.

### EXAMPLES

### Example 1: Role of snapalysin in colloidal stabilization and foam stability of beer (2L brewing trial).

The objective was to investigate the effect of snapalysin in the colloidal stabilisation of beer

The snapalysin of SEQ ID NO: 1 was used in this example. It was produced using standard recombinant techniques.

### Prepartion of the wort:

In pilot scale, mashing trials with 100 % malt were executed. Following the mashing profile in Table 1, 200L total wort volume using 54.7 kg malt were produced. Two times 2L wort were separated after lautering and cooled to 50 °C. Thereafter one aliquot of the wort was incubated with snapalysin in an amount corresponding to 1.6 mg EP (Enzyme Protein)/Litre for 1 hour. The control was also kept for 1 hour at 50°C without enzyme treatment.

**Table 1: mashing profile**

| Time (min) | Temp (°C) |
|---|---|
| 0 | 60 |
| 5 | 64 |
| 50 | 64 |
| 60 | 72 |
| 70 | 72 |
| 75 | 78 |
| 80 | 78 |

After the enzyme treatment, the wort was boiled and thereafter cooled to 15 °C where it was fermented using yeast for 7 days at 15°C. Finally the beer was filtered.

The haze was measured using the method described below.

200 microliter wort (filtered) or beer (degassed and pH adjusted (pH 4.0)) was added to a microtiter plate. A measurement was done (PolarStar OPTIMA plate reader, A550nm) to determine the background value. 100 microliter Brewtan C (200 mg/L) was then added to make up the volume to 300 microliter. The sample was analyzed with the plate reader every 2 min for a total of 16 minutes. The background value was subtracted from the measurement. The resulting value is a measurement of the content of haze sensitive proteins in the beer. The analysis was made with quadruple samples and then an average was calculated from the four measurements.

The results in EBC units are given in table below.

| **Time (min)** | 0 | 2 | 4 | 6 | 8 | 10 | 12 | 14 | 16 |
|---|---|---|---|---|---|---|---|---|---|
| **Control (without enzyme)** | 0.000 | 0.267 | 0.382 | 0.426 | 0.449 | 0.466 | 0.479 | 0.491 | 0.493 |
| **snapalysin treated** | 0.000 | 0.141 | 0.199 | 0.217 | 0.227 | 0.239 | 0.253 | 0.266 | 0.271 |

The haze in beer was also measured by a modified method according to the method published by Siebert 1997 (J. Am. Soc. Brew. Chem. 55(2):73-78, 1997). In brief, 100 ml beer (degassed and pH adjusted (pH 4.0)) and 100 ml water were added in a glass with a magnetic stirrer and the amount of background haze value was determined with a Haze-meter (Haze-meter HZ-013, Lg-automatic APS). 2x3.75 ml of 200 mg/L Brewtan C was added to the sample during stirring, and the sample was incubated at room temperature for 40 minutes before measuring the developed haze. The background value was then subtracted from the measurement after 40 minutes to give a measure of the potential of forming haze in the final fermented beer

The results are given in the table below

| | **Control (without enzyme)** | **snapalysin treated** |
|---|---|---|
| **EBC** | 9.70 | 5.03 |

From the above table, it is evident that snapalysin had good reduction of haze compared to an untreated control.

The foam stability was measured as follows:

200 ml beer (degassed and pH adjusted (pH 4.0)) was added to a foam tower. Nitrogen was bubbled through a glass filter in the foam tower. The foam formed by the bubbling of nitrogen was then transported in tubes to a graded (ml) collector funnel which was resting on a scale. The graded funnel enables the measurement of collapsed beer volume. A scale on which the funnel is resting enables the resulting foam weight to be recorded. The analysis gave the weight of formed foam (g). The total weight (g) of the foam was put against the time (min) in a graph.

The results are given in the table below:

| **Time (Min)** | **Control (without treated enzyme) (g)** | **snapalysin treated (g)** |
|---|---|---|
| 0 | 0.00 | 0.00 |
| 10 | 3.30 | 3.60 |
| 15 | 10.50 | 12.24 |
| 20 | 17.70 | 21.17 |
| 25 | 25.00 | 29.96 |
| 30 | | 38.44 |
| 35 | 39.62 | 46.65 |
| 40 | 46.64 | 54.60 |
| 45 | 53.37 | 62.20 |
| 50 | 60.70 | 69.44 |
| 55 | 67.26 | 76.11 |
| 60 | 74.00 | 82.18 |
| 65 | 79.03 | 86.70 |
| 70 | 83.08 | 88.91 |
| 75 | 86.20 | 89.83 |
| 80 | 88.33 | 90.05 |
| 85 | 88.95 | 89.77 |

From above table, it is evident that Snapalysin has no effect on the foam stability.

### Example 2: Effect of increased concentration of Snapalysin of SEQ ID NO: 1 on colloidal stability and foam stability (2L brewing trial).

The same procedures as in example 1 were repeated, except that the wort incubation was done with Snapalysin in concentration of 3.2 mg enzyme protein (EP)/litre wort. The results of colloidal and foam stability are given in tables below

### Haze results with haze-meter:

| | **Control (without enzyme)** | | | | **Snapalysin (3.2 mg/L) treated** | | | | |
|---|---|---|---|---|---|---|---|---|---|
| **EBC** | 7.29 | | | | 4.23 | | | | |

| **Time (min)** | 0 | 2 | 4 | 6 | 8 | 10 | 12 | 14 | 16 |
|---|---|---|---|---|---|---|---|---|---|
| **Control (without enzyme)** | 0.000 | 0.123 | 0.207 | 0.245 | 0.276 | 0.299 | 0.320 | 0.336 | 0.353 |
| **Snapalysin (3.2 mg/L) treated** | 0.000 | 0.057 | 0.098 | 0.117 | 0.132 | 0.146 | 0.157 | 0.168 | 0.178 |

The foam was analysed as described previously and the values are given below:

| **Time (Min)** | **Control (without enzyme)** | **Snapalysin (3.2 mg/L) treated** |
|---|---|---|
| 0 | 0.00 | 0.00 |
| 10 | | 3.73 |
| 15 | | 10.60 |
| 20 | | 18.71 |
| 25 | 21.62 | 26.8 |
| 30 | 29.80 | 34.40 |
| 35 | | 41.81 |
| 40 | 40.63 | 48.58 |
| 45 | 45.80 | 54.07 |
| 50 | 50.06 | 58.00 |
| 55 | | |
| 60 | | 61.60 |
| 65 | 56.60 | 62.20 |
| 70 | 57.36 | 62.44 |
| 75 | 57.61 | |

Similar to Example 1, Snapalysin showed again a good effect on haze reduction without harming the foam.

### Example 3: Effect of Snapalysin on colloidal stability and foam stability of beer from a 10L pilot brew.

A 10L pilot brew was made with Snapalysin in addition to the 2L brewing trials to analyse the effect of Snapalysin on colloidal stability and foam stability using different methods for colloidal stability and foam stability, respectively (see material and methods above). Wort was adjusted to pH 6 and tempered to 50°C. Snapalysin was added in a concentration of 3.2 mg enzyme protein/Litre wort and incubated for one hour

Colloidal stability was measured in the beers by forced aging (0/40/0°C):

| | **Control (without enzyme)** | **Snapalysin (3.2 mg/L) treated** |
|---|---|---|
| **Forced aging (warm days)** | 5 | 11 |

Foam stability was analysed by NIBEM:

| | **Control (without enzyme)** | **Snapalysin (3.2 mg/L) treated** |
|---|---|---|
| **NIBEM (sec)** | 261 | 274 |

From the analysis, it can be seen that the beer from Snapalysin treatment of wort resulted in a stability of 11 warm days (EBC < 2), whereas the control was only stable for 5 warm days (EBC < 2). No effect on foam stability was observed after treatment with Snapalysin. In fact, the foam after enzyme treatment looked slightly more stable than the control.

We also measured haze in the same beers as above by the method with Brewtan C and a hazemeter for determining the haze:

| | **Control (without enzyme)** | **Snapalysin (3.2 mg/L) treated** |
|---|---|---|
| **EBC** | 8.42 | 5.83 |

The amount of haze was reduced after treatment with Snapalysin further supporting an improved colloidal stability obtained after treatment with Snapalysin.

### Example 4: Effect of snapalysin from Kribbella flavida on colloidal stability and foam stability of beer (2L brewing trial).

The snapalysin from *Kribbella flavida* having SEQ ID NO: 2 was made using recombinant methods.

The wort and beer were prepared using the methods described above and the beer haze was analysed as described. 3.2 mg of the Snapalysin Enzyme Protein (EP)/litre of wort was used. The incubation temperature was 50 C and pH 6. The results are given in table below

Haze results with haze-meter:

| | **Control (without enzyme)** | ***Kribbella flavida* Snapalysin treated** |
|---|---|---|
| **EBC** | 14.75 | 5.60 |

Haze results with plate reader:

| | | | | | | | | | |
|---|---|---|---|---|---|---|---|---|---|
| **Time (min)** | 0 | 2 | 4 | 6 | 8 | 10 | 12 | 14 | 16 |
| **Control (without enzyme) (EBC)** | 0.000 | 0.140 | 0.181 | 0.193 | 0.201 | 0.207 | 0.207 | 0.210 | 0.218 |
| ***Kribbella flavida* snapalysin treated (EBC)** | 0.000 | 0.099 | 0.131 | 0.139 | 0.144 | 0.147 | 0.149 | 0.149 | 0.147 |

The foam was analysed as described previously and the values are given below:

| **Time (Min)** | **Control (without enzyme) (g)** | **Kribbella flavida snapalysin treated (g)** |
|---|---|---|
| 0 | 0 | 0 |
| 10 | 3.4 | 5.36 |
| 15 | 11.18 | |
| 20 | | 19.61 |
| 25 | 26.5 | 26.05 |
| 30 | 32.96 | |
| 35 | | 35.9 |
| 40 | | 39.37 |
| 45 | | 41.91 |
| 50 | 49.61 | 43.64 |
| 55 | 51.46 | 44.79 |
| 60 | 52.51 | 45.5 |
| 65 | 52.94 | 45.93 |
| 70 | | 46.12 |

From the haze and foam analysis results it is apparent that the *Kribbella flavida* Snapalysin reduces haze thereby improving colloidal stability and it has no significant effect on the foam stability.

### Example 5: Effect of some commercially available proteases on colloidal and foam stability of beer (2L brewing trials).

Novoren™, Esperase™, Everlase™, Relase™, Neutrase™ and Savinase™ are commercially available proteases sourced from Novozymes A/S (Bagsvaerd, Denmark).

The following concentrations of enzymes and incubation temperatures were used

| Enzyme | Concentration (mg EP/L) | Incubation temperature °C |
|---|---|---|
| Novoren | 10.0 | 70 |
| Esperase | 1.6 | 75 |
| Everlase | 1.6 | 75 |
| Relase | 1.6 | 75 |
| Savinase | 1.6 | 75 |
| Neutrase | 0.8 | 50 |

The results of the haze assays are given below

Haze results with haze-meter:

| | Novoren | | Esperase | | Everlase | | Relase | | Savinase | | Neutrase | |
|---|---|---|---|---|---|---|---|---|---|---|---|---|
| | Control | Treatment | Control | Treatment | Control | Treatment | Control | Treatment | Control | Treatment | Control | Treatment |
| EBC | 2.87 | 2.74 | 7.12 | 6.44 | 7.12 | 6.78 | 7.12 | 6.48 | 7.12 | 6.50 | 4.19 | 3.42 |

Haze results with plate reader:

| Time (min) | Novoren | | Esperase | | Everlase | | Relase | | Savinase | | Neutrase | |
|---|---|---|---|---|---|---|---|---|---|---|---|---|
| | Control | Treatment | Control | Treatment | Control | Treatment | Control | Treatment | Control | Treatment | Control | Treatment |
| 0 | 0.000 | 0.000 | 0.000 | 0.000 | 0.000 | 0.000 | 0.000 | 0.000 | 0.000 | 0.000 | 0.000 | 0.000 |
| 2 | 0.022 | 0.022 | 0.077 | 0.019 | 0.077 | 0.070 | 0.077 | 0.030 | 0.077 | 0.023 | 0.184 | 0.154 |
| 4 | 0.055 | 0.063 | 0.160 | 0.086 | 0.160 | 0.147 | 0.160 | 0.086 | 0.160 | 0.087 | 0.298 | 0.213 |
| 6 | 0.069 | 0.079 | 0.192 | 0.105 | 0.192 | 0.172 | 0.192 | 0,100 | 0.192 | 0.102 | 0.332 | 0.234 |
| 8 | 0.079 | 0.092 | 0.215 | 0.115 | 0.215 | 0.188 | 0.215 | 0.108 | 0.215 | 0.109 | 0.354 | 0.245 |
| 10 | 0.088 | 0.101 | 0.232 | 0.124 | 0.232 | 0.198 | 0.232 | 0.116 | 0.232 | 0.117 | 0.372 | 0.255 |
| 12 | 0.095 | 0.108 | 0.245 | 0.128 | 0.245 | 0.207 | 0.245 | 0.121 | 0.245 | 0.122 | 0.383 | 0.257 |
| 14 | 0.102 | 0.115 | 0.255 | 0.132 | 0.255 | 0.214 | 0.255 | 0.124 | 0.255 | 0.124 | 0.391 | 0.265 |
| 16 | 0.108 | 0.120 | 0.265 | 0.135 | 0.265 | 0.220 | 0.265 | 0.125 | 0.265 | 0.128 | 0.398 | 0.263 |

The results of the foam stability assays are given below

| **Time (Min)** | Novoren | | Esperase | | Everlase | | Relase | | Savinase | | Neutrase | |
|---|---|---|---|---|---|---|---|---|---|---|---|---|
| | control | Treated | control | Treated | control | Treated | control | Treated | control | Treated | control | Treated |
| 0 | 0.00 | 0.00 | 0.00 | 0.00 | 0.00 | 0.00 | 0.00 | 0.00 | 0.00 | 0.00 | 0.00 | 0.00 |
| 10 | 2.60 | 2.40 | | 2.02 | | 1.53 | | 1.26 | | 2.05 | 1.56 | 1.82 |
| 15 | 9.28 | 6.32 | 9.12 | 6.00 | 9.12 | 5.30 | 9.12 | 5.18 | 9.12 | 6.30 | 5.98 | 6.00 |
| 20 | 16.60 | 10.68 | 14.30 | 9.75 | 14.30 | 9.27 | 14.30 | 9.12 | 14.30 | 10.05 | 11.75 | 11.07 |
| 25 | | | 19.20 | 12.62 | 19.20 | 13.30 | 19.20 | 12.35 | 19.20 | 13.11 | 18.10 | 16.32 |
| 30 | 30.66 | 16.28 | 29.18 | 14.66 | 29.18 | 17.33 | 29.18 | 14.69 | 29.18 | 15.40 | 24.94 | 21.64 |
| 35 | 37.33 | 18.32 | 32.62 | 16.10 | 32.62 | 20.90 | 32.62 | 16.38 | 32.62 | 17.00 | 32.20 | 26.87 |
| 40 | 44.20 | 19.84 | 34.97 | 17.00 | 34.97 | 23.66 | 34.97 | 17.59 | 34.97 | 18.06 | 39.44 | 31.55 |
| 45 | 49.26 | 20.95 | 36.30 | 17.50 | 36.30 | 25.50 | 36.30 | 18.38 | 36.30 | 18.75 | 46.60 | 35.40 |
| 50 | 52.68 | 21.73 | 37.51 | 17.85 | 37.51 | 26.70 | 37.51 | 19.06 | 37.51 | 19.19 | 53.34 | 38.40 |
| 55 | 55.65 | 22.19 | 37.60 | 17.90 | 37.60 | 27.46 | 37.60 | 19.43 | 37.60 | 19.39 | 59.39 | 40.53 |
| 60 | 57.38 | 22.52 | | | | 27.78 | | 19.51 | | | | 41.97 |
| 65 | 58.58 | 22.71 | | | | 27.95 | | | | | | |
| 70 | 59.18 | 22.85 | | | | | | | | | | |
| 75 | 59.20 | | | | | | | | | | | |

From the above results it is apparent that Novoren has no effect on haze (colloidal stability) while it negatively affects foam stability. Esperase, Relase, Savinase and Neutrase have positive effect on colloidal stability but also negatively affect the foam. The protease Everlase has only minor effect on colloidal stability and negative effect on foam stability.

### Example 6: Effect of other proteases in beer stability (2L brewing trials)

An aspartic endopeptidic protease belonging to peptidase A1 family and having SEQ ID NO: 3 and a metalloprotease from *Thermoascus aurantiacus* of SEQ ID NO: 4 was synthesised using standard recombinant techniques.

The following concentrations of enzymes and incubation temperatures were used

| Enzyme | Concentration (mg EP/L) | Incubation temperature C |
|---|---|---|
| Protease SEQ ID NO: 3 | 10.0 | 50 |
| Protease SEQ ID NO: 4 | 0.8 | 70 |

The results of the haze assays are given below

Haze results with haze-meter:

| | Protease SEQ ID NO: 3 | | Protease SEQ ID NO: 4 | |
|---|---|---|---|---|
| | Control | Treatment | Control | Treatment |
| EBC | 2.87 | 2.64 | 4.19 | 3.68 |

Haze results with plate reader:

| Time (min) | Protease SEQ ID NO: 3 | | Protease SEQ ID NO: 4 | |
|---|---|---|---|---|
| | Control | Treatment | Control | Treatment |
| 0 | 0.000 | 0.000 | 0.000 | 0.000 |
| 2 | 0.022 | 0.017 | 0.184 | 0.173 |
| 4 | 0.055 | 0.045 | 0.298 | 0.241 |
| 6 | 0.069 | 0.057 | 0.332 | 0.263 |
| 8 | 0.079 | 0.066 | 0.354 | 0.276 |
| 10 | 0.088 | 0.073 | 0.372 | 0.281 |
| 12 | 0.095 | 0.079 | 0.383 | 0.289 |
| 14 | 0.102 | 0.085 | 0.391 | 0.292 |
| 16 | 0.108 | 0.090 | 0.398 | 0.299 |

The results of the foam stability assays are given below

| **Time (Min)** | Protease SEQ ID NO: 3 | | Protease SEQ ID NO: 4 | |
|---|---|---|---|---|
| | control | Treated | control | Treated |
| 0 | 0.00 | 0.00 | 0.00 | 0.00 |
| 10 | 2.60 | 2.40 | 1.56 | 1.90 |
| 15 | 9.28 | 9.25 | 5.98 | 5.68 |
| 20 | 16.60 | 16.70 | 11.75 | 10.15 |
| 25 | | | 18.10 | 15.23 |
| 30 | 30.66 | 28.22 | 24.94 | 20.65 |
| 35 | 37.33 | 33.4 | 32.20 | 25.77 |
| 40 | 44.20 | 38.00 | 39.44 | 30.06 |
| 45 | 49.26 | 41.61 | 46.60 | 33.46 |
| 50 | 52.68 | 44.49 | 53.34 | 35.95 |
| 55 | 55.65 | 46.40 | 59.39 | 37.63 |
| 60 | 57.38 | 47.77 | | 38.77 |
| 65 | 58.58 | 48.69 | | |
| 70 | 59.18 | 49.14 | | |
| 75 | 59.20 | 49.50 | | |

From the above results, it is apparent that Protease of SEQ ID NO: 3 has no or minor effect on both colloidal stability and foam stability while Protease of SEQ ID NO:4 has positive effect on colloidal stability (i.e. reduces haze) but negatively affects the foam.

The invention described and claimed herein is not to be limited in scope by the specific aspects herein disclosed, since these aspects are intended as illustrations of several aspects of the invention. Any equivalent aspects are intended to be within the scope of this invention. Indeed, various modifications of the invention in addition to those shown and described herein will become apparent to those skilled in the art from the foregoing description. Such modifications are also intended to fall within the scope of the appended claims. In the case of conflict, the present disclosure including definitions will control.

## Claims

1. A method of improving colloidal stability in beer comprising contacting a mash and/or a wort with a snapalysin during the production of beer.

2. The method according to claim 1, wherein the snapalysin is added to the wort.

3. The method according to any of the preceding claims, wherein the snapalysin is obtainable from Actinomycetales.

4. The method according to claim 3, wherein the Actinomycetale is *Streptomyces.*

5. The method according to claim 3, wherein the Actinomycetale is *Kribbella.*

6. The method according to any of the preceding claims, wherein the contacting is done from 5 minutes to 120 minutes.

7. The method according to any of the preceding claims, wherein 0.01 to 100 mg snapalysin EP (Enzyme Protein) per kg malt is used.

8. The method according to any of the preceding claims, wherein 0.0016 to 16 mg snapalysin EP per litre wort is used.

9. The method according to any of the preceding claims, wherein the colloidal stability is increased by at least 10% compared to a beer brewed in the absence of snapalysin.

10. The method according to any of the preceding claims, wherein the foam stability is at least 80% compared to a beer brewed in the absence of snapalysin.

11. The method according to any of the preceding claims, wherein the beer is produced without processing aids.

12. The method according to any of the preceding claims, wherein the beer is produced without stabilization with silica gel.

13. The method according to any of the preceding claims, wherein the contacting is done after lautering.

14. The method according to any of the preceding claims, wherein the contacting is done during sparging.

15. The method according to any of the preceding claims, wherein the contacting is done at a temperature in the range of from 20 to 80°C.

16. The method according to any of the preceding claims, wherein the contacting is done at a temperature of at least 50°C.

17. Use of snapalysin in brewing of beer.

## Patentansprüche

1. Verfahren zum Verbessern der kolloidalen Stabilität in Bier, umfassend das In-Kontakt-Bringen einer Maische und/oder einer Würze mit einem Snapalysin während der Herstellung von Bier.

2. Verfahren nach Anspruch 1, wobei das Snapalysin zu der Würze hinzugefügt wird.

3. Verfahren nach einem beliebigen der vorhergehenden Ansprüche, wobei das Snapalysin aus *Actinomycetalen* erhältlich ist.

4. Verfahren nach Anspruch 3, wobei die *Actinomycetale Streptomyces* ist.

5. Verfahren nach Anspruch 3, wobei die *Actinomycetale Kribella* ist.

6. Verfahren nach einem beliebigen der vorhergehenden Ansprüche, wobei das In-Kontakt-Bringen von 5 Minuten bis 120 Minuten erfolgt.

7. Verfahren nach einem beliebigen der vorhergehenden Ansprüche, wobei 0,01 bis 100 mg Snapalysin EP (Enzymprotein) pro kg Malz verwendet wird.

8. Verfahren nach einem beliebigen der vorhergehenden Ansprüche, wobei 0,0016 bis 16 mg Snapalysin EP pro Liter Würze verwendet wird.

9. Verfahren nach einem beliebigen der vorhergehenden Ansprüche, wobei die kolloidale Stabilität um mindestens 10% im Vergleich zu einem Bier, das in der Abwesenheit von Snapalysin gebraut wurde, erhöht ist.

10. Verfahren nach einem beliebigen der vorhergehenden Ansprüche, wobei die Schaumstabilität mindestens 80% im Vergleich zu einem Bier, das in der Abwesenheit von Snapalysin gebraut wurde, beträgt.

11. Verfahren nach einem beliebigen der vorhergehenden Ansprüche, wobei das Bier ohne Verarbeitungshilfsstoffe hergestellt wird.

12. Verfahren nach einem beliebigen der vorhergehenden Ansprüche, wobei das Bier ohne Stabilisierung mit Kieselgel hergestellt wird.

13. Verfahren nach einem beliebigen der vorhergehenden Ansprüche, wobei das In-Kontakt-Bringen nach dem Läutern durchgeführt wird.

14. Verfahren nach einem beliebigen der vorhergehenden Ansprüche, wobei das In-Kontakt-Bringen beim Anschwänzen durchgeführt wird.

15. Verfahren nach einem beliebigen der vorhergehenden Ansprüche, wobei das In-Kontakt-Bringen bei einer Temperatur im Bereich von 20 bis 80 °C erfolgt.

16. Verfahren nach einem beliebigen der vorhergehenden Ansprüche, wobei das In-Kontakt-Bringen bei einer Temperatur von mindestens 50 °C erfolgt.

17. Verwendung von Snapalysin beim Brauen von Bier.

## Revendications

1. Méthode pour améliorer la stabilité colloïdale dans la bière comprenant la mise en contact d'un brassin et/ou d'un moût avec une snapalysine pendant la production de la bière.

2. Méthode selon la revendication 1, dans laquelle la snapalysine est ajoutée au moût.

3. Méthode selon l'une quelconque des revendications précédentes, dans laquelle la snapalysine peut être obtenue à partir d'Actinomycetales.

4. Méthode selon la revendication 3, dans laquelle l'Actinomycetale est *Streptomyces.*

5. Méthode selon la revendication 3, dans laquelle l'Actinomycetale est *Kribbella.*

6. Méthode selon l'une quelconque des revendications précédentes, dans laquelle la mise en contact est effectuée pendant 5 à 120 minutes.

7. Méthode selon l'une quelconque des revendications précédentes, dans laquelle on utilise de 0,01 à 100 mg de EP (protéine enzymatique) de snapalysine par kg de malt.

8. Méthode selon l'une quelconque des revendications précédentes, dans laquelle on utilise de 0,0016 à 16 mg de EP de snapalysine par kg de moût.

9. Méthode selon l'une quelconque des revendications précédentes, dans laquelle la stabilité colloïdale est augmentée d'au moins 10 % comparativement à une bière brassée en l'absence de snapalysine.

10. Méthode selon l'une quelconque des revendications précédentes, dans laquelle la stabilité de la mousse est d'au moins 80 % comparativement à une bière brassée en l'absence de snapalysine.

11. Méthode selon l'une quelconque des revendications précédentes, dans laquelle la bière est produite sans auxiliaires technologiques.

12. Méthode selon l'une quelconque des revendications précédentes, dans laquelle la bière est produite sans stabilisation avec un gel de silice.

13. Méthode selon l'une quelconque des revendications précédentes, dans laquelle la mise en contact est effectuée après la filtration du moût.

14. Méthode selon l'une quelconque des revendications précédentes, dans laquelle la mise en contact est effectuée pendant le lavage des drêches.

15. Méthode selon l'une quelconque des revendications précédentes, dans laquelle la mise en contact est effectuée à une température dans la plage de 20 à 80°C.

16. Méthode selon l'une quelconque des revendications précédentes, dans laquelle la mise en contact est effectuée à une température d'au moins 50°C.

17. Utilisation de la snapalysine dans le brassage de la bière.
